# EUROPÄISCHE PATENTANMELDUNG

(11) **EP 1 070 693 A2**
(43) Veröffentlichungstag der Anmeldung: **24.01.2001**
(21) Anmeldenummer: 00115592.8
(22) Anmeldetag: 19.07.2000
(51) Int. Cl.: C07C 2/10, C07C 11/02, C07C 29/16, C07C 41/03, C07C 43/13, C08K 5/12, C08L 27/06

(54) **Verfahren zur Herstellung von Alkoholgemischen**

(30) Priorität: 20.07.1999 DE 19933827
(71) Anmelder: BASF AKTIENGESELLSCHAFT, 67056 Ludwigshafen (DE)
(72) Erfinder: Maas, Heiko, 68165 Mannheim (DE); Schwab, Peter, 67098 Bad Dürkheim (DE); Breitscheidel, Boris, 67117 Limburgerhof (DE)
(74) Vertreter: Kinzebach, Werner, Dr.

(57) **Zusammenfassung**

Die vorliegende Erfindung betrifft ein Verfahren zur Herstellung von Alkoholgemischen, wobei man
a) ein Kohlenwasserstoffgemisch, das Cyclopenten und wenigstens ein acyclisches Monoolefin enthält, in einer Metathesereaktion umsetzt,
b) aus dem Reaktionsgemisch der Metathese eine Olefinfraktion isoliert, die im Wesentlichen Olefine mit 6 bis 12 Kohlenstoffatomen enthält,
c) gegebenenfalls die in der Olefinfraktion enthaltenden zwei- oder mehrfach ungesättigten Verbindungen zumindest teilweise einer selektiven Hydrierung zu Monoolefinen unterwirft, und
d) die, gegebenenfalls selektiv hydrierte, Olefinfraktion durch Umsetzung mit Kohlenmonoxid und Wasserstoff katalytisch hydroformyliert und hydriert,
Alkoholgemische, die durch ein solches Verfahren erhältlich sind, ein Verfahren zur Veresterung der Alkoholgemische, die so erhaltenen Ester und deren Verwendung.

## Beschreibung

Die vorliegende Erfindung betrifft ein Verfahren zur Herstellung von Alkoholgemischen und die nach diesem Verfahren erhaltenen Gemische. Sie betrifft weiter ein Verfahren zur Veresterung dieser Alkoholgemische, die so erhaltenen Ester und deren Verwendung als Weichmacher.

Zur Modifizierung der thermoplastischen Eigenschaften einer Vielzahl grosstechnisch wichtiger Produkt, wie Z.B. Kunststoffe, Lacke, Anstrich- und Beschichtungsmittel, Dichtungsmassen, Kautschuk- und Gummi-Artikel, werden in grossen Mengen sogenannte Weichmacher eingesetzt. Dabei werden an die Weichmacher in Abhängigkeit vom jeweiligen Anwendungsbereich eine Reihe von Ansprüchen gestellt. Allgemein sollen diese z.B. im Wesentlichen geruchlos, licht-, kälte- und wärmebeständig, nicht gesundheitsschädlich und möglichst wenig flüchtig sein. Wichtige Eigenschaften von Weichmachern gegenüber Polymeren sind Verträglichkeit, Geliervermögen und weichmachende Wirksamkeit.

Eine wichtige Klasse von Weichmachern sind die Ester-Weichmacher, zu denen unter Anderem Phthalsäureester, Trimellithsäureester, acyclische aliphatische Dicarbonsäureester, wie die Ester der Adipinsäure, Sebacinsäure, Azelainsäure etc., Phosphorsäureester, Fettsäureester und Hydroxycarbonsäureester zählen. Von besonderer Bedeutung sind die Phthalate, insbesondere Dioctylphthalat, Di-(2-ethylhexyl)phthalat und Diisodecylphthalat, die z.B. zur Herstellung von Weich-PVC eingesetzt werden.

Die zur Herstellung der Ester-Weichmacher eingesetzten Alkohole werden allgemein als Weichmacheralkohole bezeichnet. Eine wichtige Klasse der Weichmacheralkohole sind überwiegend lineare C₆- bis C₁₁-Alkohole. So weisen z.B. Phthalate auf Basis dieser Alkohole im Allgemeinen gute anwendungstechnische Eigenschaften , wie z.B. eine hohe Kälteelastizität und eine niedrige Flüchtigkeit auf. Dabei werden die weichmachenden Eigenschaften der Ester-Weichmacher unter Anderem von der Kettenlänge und vom Verzweigungsgrad der Alkoholkomponente beeinflusst. So weisen Phthalsäureester auf Basis kürzerkettiger Alkohole im Allgemeinen eine gute Gelierkraft auf, sind aber in Bezug auf ihre Flüchtigkeit verbesserungswürdig. Phthalsäureester auf Basis längerkettiger Alkohole weisen zwar im Allgemeinen eine erniedrigte Flüchtigkeit auf, die jedoch häufig mit einer schlechteren Gelierkraft und einer schlechteren Kältebeständigkeit verbunden ist. Eine Verbesserung der Kälteflexibilität kann häufig durch den Einsatz von Alkoholen erzielt werden, die einen gewissen Grad an Kettenverzweigungen aufweisen.

Zur Herstellung vom Ester-Weichmachern mit verbesserten anwendungstechnischen Eigenschaften besteht ein Bedarf an Weichmacheralkoholen mit etwa 6 bis 12 Kohlenstoffatomen, die zu einem geringen Grad verzweigt sind (sogenannte semilineare Alkohole), und an entsprechenden Gemischen davon.

Alkohole mit etwa 6 bis 12 Kohlenstoffatomen sind sowohl aus nativen Quellen als auch auf synthetischem Weg, z.B. durch Aufbau aus Edukten mit einer geringeren Zahl an Kohlenstoffatomen erhältlich. So erhält man z.B. nach dem SHOP-Prozess (Shell higher olefine process) oder dem Chevron-Verfahren, ausgehend von Ethen, Olefinfraktionen mit einer für die Weiterverarbeitung zu Weichmacheralkoholen geeigneten Kohlenstoffanzahl. Die Funktionalisierung der Olefine zu den entsprechenden Alkoholen erfolgt dabei z.B. durch Hydroformylierung und Hydrierung, wobei je nach Reaktionsführung einstufig oder in zwei separaten Reaktionsstufen gearbeitet werden kann. Eine Übersicht von Hydroformylierungsverfahren und geeigneten Katalysatoren findet sich in Beller et al. Journal of Molecular Catalysis A 104 (1995), S. 17-85. Nachteilig an den auf Ethylen basierenden Verfahren zur Herstellung von Alkoholen sind die hohen Kosten des Ausgangsmaterials, wodurch diese Verfahren wirtschaftlich benachteiligt sind.

Bei der Aufarbeitung von Erdöl durch Steamcracken fällt unter anderem ein als C₅-Schnitt bezeichnetes Kohlenwasserstoffgemisch mit einem Gesamtolefinanteil von z.B. etwa 50% an, von denen ca. 15% auf Cyclopenten und der Rest auf acyclische Monoolefine, vor allem n-Penten (ca. 15 Gew.-%) und weitere isomere Methylbutene (ca. 20 Gew.-%) entfallen. Bislang erfolgte die großtechnische Aufarbeitung des C₅-Schnittes im Wesentlichen destillativ zur Gewinnung des darin enthaltenen Cyclopentans. Derartige Verfahren sind verfahrenstechnisch sehr aufwendig. Es besteht daher ein Bedarf zur nicht-destillativen Entfernung des Cyclopentens und gegebenenfalls weiterer acyclischer Monoolefine aus dem C₅-Schnitt unter Gewinnung von Wertprodukten.

Die DE-A-196 54 166 beschreibt Oligomerengemische mit ethylenisch ungesättigten Doppelbindungen, die von Cyclopenten abgeleitet sind und die durch Metathesereaktion eines C₅-Schnittes in Gegenwart eines Übergangsmetallkatalysators erhalten werden.

Die DE-A-196 54 167 beschreibt ein Verfahren zur Funktionalisierung solcher Oligomerengemische, die von Cyclopenten abgeleitet sind, z.B. durch Hydroformylierung und gegebenenfalls anschliessende Hydrierung. Dabei resultieren Alkoholgemische mit einem hohen Anteil an zwei- und höherwertigen Alkoholen. Derartige Alkoholgemische eignen sich nicht für einen Einsatz als Weichmacheralkohole.

Der vorliegenden Erfindung liegt die Aufgabe zugrunde, ein Verfahren zur Herstellung von Alkoholen zur Verfügung zu stellen. Diese sollen sich vorzugsweise als Weichmacheralkohole zur Herstellung von Ester-Weichmachern eignen. Insbesondere soll bei der Herstellung dieser Alkohole möglichst auf einen Einsatz von Edukten mit hohen Produktionskosten, wie insbesondere Ethen, verzichtet werden. Vorzugsweise soll in dem erfindungsgemässen Verfahren ein grosstechnisch anfallendes Ausgangskohlenwasserstoffgemisch eingesetzt werden.

Überraschenderweise wurde nun gefunden, daß die Aufgabe durch ein Verfahren gelöst wird, bei dem man ein Kohlenwasserstoffgemisch, das Cyclopenten und wenigstens ein acyclisches Monoolefin enthält, einer Metathese unterzieht, aus dem Metathesegemisch eine C₆-C₁₂-Olefinfraktion isoliert und diese anschliessend einer katalytischen Hydroformylierung und Hydrierung unterzieht.

Gegenstand der Erfindung ist somit ein Verfahren zur Herstellung von Alkoholgemischen, wobei man
a) ein Kohlenwasserstoffgemisch, das Cyclopenten und wenigstens ein acyclisches Monoolefin enthält, in einer Metathesereaktion umsetzt,
b) aus dem Reaktionsgemisch der Metathese eine Olefinfraktion isoliert, die im Wesentlichen Olefine mit 6 bis 12 Kohlenstoffatomen enthält,
c) gegebenenfalls die in der Olefinfraktion enthaltenen zwei- oder mehrfach ungesättigten Verbindungen zumindest teilweise einer selektiven Hydrierung zu Monoolefinen unterwirft, und
d) die, gegebenenfalls selektiv hydrierte, Olefinfraktion durch Umsetzung mit Kohlenmonoxid und Wasserstoff katalytisch hydroformyliert und hydriert.

### Schritt a)

Bevorzugt wird für die Metathesereaktion ein Kohlenwasserstoffgemisch mit einem Cyclopentengesamtgehalt im Bereich von etwa 5 bis 40 Gew.-%, bevorzugter 10 bis 30 Gew.-%, insbesondere 12 Gew.-% bis 20 Gew.-%, eingesetzt.

Bevorzugt beträgt der Gesamtolefingehalt des zur Metathese eingesetzten Kohlenwasserstoffgemisches mindestens 30 Gew.-%, bevorzugt mindestens 40 Gew.-%, insbesondere mindestens 50 Gew.-%. Geeignet sind Kohlenwasserstoffgemische mit einem Gesamtolefingehalt von bis zu 100 Gew.-%.

Vorzugsweise enthalten die zur Metathese eingesetzten Kohlenwasserstoffgemische wenigstens ein acyclisches Monoolefin. Bevorzugte acyclische Monoolefine sind ausgewählt unter C₅-Monoolefinen, wie 1-Penten, 2-Penten, 2-Methyl-1-buten, 3-Methyl-1-buten, 2-Methyl-2-buten und Gemischen davon. Bevorzugt beträgt der Anteil von C₅-Monoolefinen an den acyclischen Monoolefinen mindestens 70 Gew.-%, bevorzugt mindestens 80 Gew.-%, insbesondere mindestens 90 Gew.-%.

Vorzugsweise wird für die Metathesereaktion in Schritt a) ein bei der Erdölverarbeitung großtechnisch anfallendes Kohlenwasserstoffgemisch verwendet. Derartige Gemische können gewünschtenfalls zuvor einer katalytischen Teilhydrierung zur Entfernung von Dienen unterzogen werden. Besonders geeignet ist z.B. ein an gesättigten und ungesättigten C₅-Kohlenwasserstoffen angereichertes Gemisch, insbesondere ein C₅-Schnitt. Zur Gewinnung eines für den Einsatz in dem erfindungsgemäßen Verfahren geeigneten C₅-Schnittes eignet sich vorzugsweise Pyrolysebenzin, das Z.B. beim Steamcracken von Naphtha anfällt. Dieses Pyrolysebenzin kann gewünschtenfalls zuerst einer Selektivhydrierung unterzogen werden, um enthaltene Diene und Acetylene im Wesentlichen selektiv in die entsprechenden Alkane und Alkene zu überführen. Anschließend erfolgt ein Trennschritt, z.B. eine fraktionierte Destillation, wobei zum einen der für weitere chemische Synthesen wichtige C₆-C₈-Schnitt, der die aromatischen Kohlenwasserstoffe enthält und zum anderen ein in Schritt a) des erfindungsgemässen Verfahrens einsetzbarer C₅-Schnitt anfallen.

Bevorzugt wird zur Metathese ein großtechnisch anfallender C₅-Schnitt mit einem Gesamtolefinanteil von z.B. 50 bis 60 Gew.-%, wie etwa 56%, einem Cyclopentenanteil von z.B. 10 bis 20 Gew.-%, wie etwa 15 Gew.-% und einem Anteil an C₅-Monoolefinen von z.B. 33 bis 43 Gew.-%, wie etwa 38 Gew.-%, eingesetzt. Dabei entfallen bevorzugt etwa 16 Gew.-% auf das n-Penten und etwa 22 Gew.-% auf isomere Pentene.

In Schritt a) des erfindungsgemässen Verfahrens kann auch vorteilhaft ein Kohlenwasserstoffgemisch verwendet werden, welches ein Cyclopenten-haltiges Kohlenwasserstoffgemisch, insbesondere den C₅-Schnitt, und ein acyclische C₄-Olefine enthaltendes Gemisch umfasst. Bevorzugt handelt es sich bei dem C₄-Olefingemisch um eine Erdölfraktion, insbesondere um Raffinat II. Raffinat II ist z.B. durch Cracken hochmolekularer Kohlenwasserstoffe, wie Rohöl, erhältlich. Bevorzugt wird ein C₄-Olefingemisch eingesetzt, das 60 bis 85 Vol.-% Buten-1 und Buten-2 enthält. Vorzugsweise enthält das C₄-Olefingemisch höchstens 40 Vol.-%, bevorzugt höchstens 20 Vol.-%, gesättigte Kohlenwasserstoffe, wie n-Butan, Isobutan, C₅-Alkane etc.

Die Metathesereaktion des Kohlenwasserstoffgemisches in Schritt a) umfaßt vorzugsweise
i) die Disproportionierung von acyclischen Monoolefinen durch Kreuzmetathese,
ii) die Oligomerisation von Cyclopenten durch ringöffnende Metathese,
iii) den Kettenabbruch durch Umsetzung eines Oligomers aus ii) mit einem acyclischen Olefin des Kohlenwasserstoffgemisches oder einem Produktes aus i),
wobei die Schritte i) und/oder ii) und/oder iii) mehrmals für sich alleine oder in Kombination durchlaufen werden können.

### Schritt i)

Durch Kombinationen aus Kreuzmetathese verschiedener und Selbstmetathese gleicher acyclischer Olefine sowie durch mehrmaliges Durchlaufen dieser Reaktion werden eine Vielzahl von Monoolefinen mit unterschiedlicher Struktur und Kohlenstoffatomanzahl erhalten, die die Endgruppen der Oligomere in dem bei der Metathese resultierenden Reaktionsgemisch bilden. Nach einer bevorzugten Ausführungsform des erfindungsgemässen Verfahrens wird für die Metathese ein Katalysator eingesetzt, der die Bildung von Kreuzmetatheseprodukten ermöglicht. Dazu zählen bevorzugt die im Folgenden als besonders aktiv beschriebenen Metathesekatalysatoren. Nach dieser Ausführungsform werden Metathesegemische mit einem höheren Anteil an Oligomeren ohne terminale Doppelbindungen erhalten, als bei Einsatz eines weniger aktiven Katalysators.

### Schritt ii)

Die durchschnittliche Anzahl an Cyclopenten-Insertionen in die wachsende Kette im Sinne einer ringöffnenden Metathesepolymerisation bestimmt das mittlere Molekulargewicht des gebildeten Cyclopenten-Oligomerengemisches. Vorzugsweise werden durch das erfindungsgemäße Verfahren Oligomerengemische mit einem mittleren Molekulargewicht in einem Bereich von etwa 138 bis 206 gebildet, was einer durchschnittlichen Anzahl von einer bis zwei Cyclopenteneinheiten pro Oligomer entspricht.

### Schritt iii)

Der Kettenabbruch erfolgt durch Umsetzung eines Oligomeren, welches noch ein aktives Kettenende in Form eines Katalysatorkomplexes (Alkylidenkomplexes) aufweist, mit einem acyclischen Olefin, wobei im Allgemeinen ein aktiver Katalysatorkomplex zurückgewonnen wird. Dabei kann das acyclische Olefin unverändert aus dem ursprünglich zur Reaktion eingesetzten Kohlenwasserstoffgemisch stammen oder zuvor in einer Kreuzmetathese nach Stufe i) modifiziert worden sein.

Geeignete Katalysatoren für die Metathese sind bekannt und umfassen homogene und heterogene Katalysatorsysteme. Im allgemeinen eignen sich für das erfindungsgemäße Verfahren Katalysatoren auf Basis eines übergangsmetalls der 6., 7. oder 8. Nebengruppe des Periodensystems, wobei vorzugsweise Katalysatoren auf Basis von Mo, W, Re und Ru verwendet werden.

Bevorzugt werden Katalysator/Cokatalysator-Systeme auf Basis von W, Mo und Re eingesetzt, die mindestens eine lösliche Übergangsmetallverbindung und/oder ein alkylierendes Agens umfassen können. Dazu zählen z.B. MoCl₂(NO)₂(PR₃)₂/Al₂(CH₃)₃Cl₃; WCl₆/BuLi; WCl₆/EtAlCl₂(Sn(CH₃)₄)/EtOH; WOCl₄/Sn(CH₃)₄; WOCl₂(O-[2,6-Br₂-C₆H₃])/Sn(CH₃)₄ und CH₃ReO₃/C₂H₅AlCl₂.

Bevorzugte Katalysatoren sind weiterhin vierfach koordinierte Mo- und W-Alkylidenkomplexe, die zusätzlich zwei sperrige Alkoxy- und einen Imido-Liganden aufweisen, insbesondere ((CH₃)₃CO)₂Mo(=N-[2,6-(i-C₃H₇)₂-C₆H₃])(=CHC(CH₃)₂C₆H₅) und [(CF₃)₂C(CH₃)O]₂Mo(=N-[2,5-(i-C₃H₇)-C₆H₃])(=CH(CH₃)₂C₆H₅).

Weiterhin bevorzugt werden homogene Metathesekatalysatoren eingesetzt, die in der Angew. Chem. 107, S. 2179 ff. (1995), in J. Am. Chem. Soc. 118, S. 100 ff. (1996) sowie in J. Chem. Soc., Chem. Commun., S. 1127 ff. (1995) beschrieben sind. Dazu zählen insbesondere Katalysatoren der allgemeinen Formel RuCl₂(=CHR)(PR'₃)₂, wie
RuCl₂(=CHCH₃)(P(C₆H₁₁)₃)₂, (η⁶-p-Cymol)RuCl₂(P(C₆H₁₁)₃)/(CH₃)₃SiCHN₂ und (η⁶-p-Cymol)RuCl₂(P(C₆H₁₁)₃)/C₆H₅CHN₂.

Ein besonders bevorzugter heterogener Katalysator ist Re₂O₇ auf Al₂O₃ als Trägermaterial.

Gewünschtenfalls können je nach verwendetem Katalysator bei der Metathese Oligomerengemische mit wechselnden Doppelbindungsanteilen und wechselnden Anteilen an terminalen Doppelbindungen erhalten werden. Vorzugsweise wird ein Katalysator mit hoher katalytischer Aktivität, wie RuCl₂(=CHC₆H₅)(P(C₆H₁₁)₃)₂ oder Re₂O₇ auf Al₂O₃ eingesetzt, wobei Metatheseprodukte mit einem möglichst geringen Anteil an Doppelbindungen und einer geringen Jodzahl erhalten werden.

Bevorzugt weisen die in Schritt a) erhaltenen Metathese-Reaktionsgemische eine Jodzahl im Bereich von etwa 200 bis 400 g I₂/100 g Oligomere auf.

Vorzugsweise resultiert bei der Metathesereaktion in Schritt a) ein Oligomerengemisch, daß im Wesentlichen von Cyclopenten abgeleitete Oligomere der allgemeinen Formel I 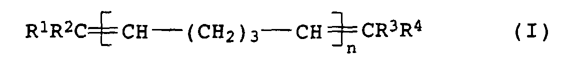 umfasst, worin
n für eine ganze Zahl von 1 bis 15 steht, und
R¹, R², R³, R⁴ unabhängig voneinander für Wasserstoff oder Alkyl stehen.

Die Reste R¹, R², R³ und R⁴ in der Formel I stehen unabhängig voneinander für Wasserstoff oder Alkyl, wobei der Ausdruck "Alkyl" geradkettige und verzweigte Alkylgruppen umfaßt.

Vorzugsweise handelt es sich dabei um geradkettige oder verzweigte C₁-C₁₅-Alkyl-, bevorzugt C₁-C₁₀-Alkyl-, insbesondere bevorzugt C₁-C₅-Alkylgruppen. Beispiele für Alkylgruppen sind insbesondere Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Di-methylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 1-Ethylpentyl, 2-Ethylpentyl, 1-Propylbutyl, Octyl, Decyl, Dodecyl, etc.

Der Wert n in der Formel I steht für die Anzahl der in die von Cyclopenten abgeleiteten Oligomerengemische durch ringöffnende Metathesereaktion eingeführten Cyclopenten-Einheiten. Der Wert n und somit der Grad der ringöffnenden Metathese kann durch die Aktivität des verwendeten Metathesekatalysators und das Verhältnis von acyclischen zu cyclischen Olefinen beeinflußt werden.

Vorzugsweise liegt der Wert für n in einem Bereich von etwa 1 bis 3, insbesondere 1 bis 2.

Bevorzugt sind Oligomerengemische der Formel I, bei denen mindestens 50 Gew.-%, insbesondere mindestens 70 Gew.-% (ermittelt durch die Flächenintegration der Gaschromatogramme) der Komponenten einen Wert von n > 1 aufweist.

Für Schritt a) des erfindungsgemässen Verfahrens geeignete Reaktionsbedingungen und Katalysatoren sind in der DE-A-196 54 166 und DE-A-196 54 167 beschrieben, auf die hier in vollem Umfang Bezug genommen wird.

### Schritt b)

Zur Isolierung einer Olefinfraktion, die im Wesentlichen Olefine mit 6 bis 12 Kohlenstoffatomen aufweist, wird Reaktionsgemisch aus Schritt a) einem oder mehreren Trennschritten unterworfen. Geeignete Trennvorrichtungen sind die üblichen, dem Fachmann bekannten Apparaturen. Dazu zählen z. B. Destillationskolonnen, z. B. Bodenkolonnen, die gewünschtenfalls mit Glocken, Siebplatten, Siebböden, Ventilen, Seitenabzügen etc. ausgerüstet sein können, Verdampfer, wie Dünnschichtverdampfer, Fallfilmverdampfer, Wischblattverdampfer, Sambay-Verdampfer etc. und Kombinationen davon. Bevorzugt erfolgt die Isolierung der Olefinfraktion durch fraktionierte Destillation.

Vorzugsweise isoliert man in Schritt b) eine Olefinfraktion, die im Wesentlichen Olefine mit 7 bis 11 Kohlenstoffatomen, bevorzugt 8 bis 10 Kohlenstoffatomen, enthält.

Vorzugsweise weist die in Schritt b) isolierte Olefinfraktion einen möglichst hohen Olefinanteil auf. Bevorzugt beträgt der Olefinanteil mindestens 40 Gew.-%, bevorzugt mindestens 50 Gew.-%. Nach einer besonders bevorzugten Ausführungsform liegt der Olefinanteil bei etwa 100 Gew.-%.

Wie zuvor beschrieben, kann zur Metathese in Schritt a) ein Kohlenwasserstoffgemisch eingesetzt werden, das neben Cyclopenten und acyclischen Monoolefinen auch gesättigte Verbindungen aufweist. Enthält die in Schritt b) isolierte Olefinfraktion einen Teil dieser gesättigten Verbindungen, so ist dies für die Weiterverarbeitung zu den erfindungsgemäßen Olefingemischen im Allgemeinen unkritisch. Vorzugsweise weist die in Schritt b) isolierte Olefinfraktion einen Anteil an gesättigten Verbindungen im Bereich von etwa 0 bis 60 Gew.-%, bevorzugt etwa 0,1 bis 50 Gew.-%, auf.

Wie zuvor beschrieben, resultieren bei der Metathese in Schritt a) im Allgemeinen Reaktionsgemische, die neben Monoolefinen und gegebenenfalls gesättigten Verbindungen aus dem Eduktkohlenwasserstoffgemisch auch zweifach und gegebenenfalls mehrfach ungesättigte Verbindungen aufweisen können. Vorzugsweise weist die in Schritt b) isolierte Olefinfraktion einen Anteil an zwei- und mehrfach ungesättigten Verbindungen im Bereich von etwa 60 bis 100 Gew.-% auf.

Gewünschtenfalls kann in Schritt b) noch wenigstens eine weitere Olefinfraktion aus dem Reaktionsgemisch der Metathese isoliert werden. Vorzugsweise handelt es sich dabei um Fraktionen, die in dem in Schritt a) erhaltenen Reaktionsgemisch in einer Menge von mindestens 5 Gew.-%, bezogen auf die Gesamtmenge des Gemisches, enthalten sind.

Bevorzugt wird in Schritt b) wenigstens eine weitere Olefinfraktion isoliert, die im Wesentlichen Olefine mit mehr als 12, bevorzugt mehr als 11, insbesondere mehr als 10 Kohlenstoffatomen enthält. Vorzugsweise enthält diese Fraktion im Wesentlichen Olefine mit höchstens 50, besonders bevorzugt höchstens 30 Kohlenstoffatomen. Zur weiteren Verarbeitung können diese Olefinfraktionen vorzugsweise einer Kreuzmetathese mit wenigsten einem acyclischen Olefin unterzogen werden, das höchstens 5, bevorzugt höchstens 4 Kohlenstoffatome aufweist. Für die Kreuzmetathese geeignete niedermolekulare Olefine und Olefingemische sind z.B. Ethen, Raffinat II oder entsprechende niedermolekulare Olefinfraktionen aus der Metathese aus Schritt a). Zur weiteren Verarbeitung können diese höhermolekularen weiteren Olefinfraktionen gewünschtenfalls auch teilweise oder vollständig erneut in Schritt a) des erfindungsgemässen Verfahrens eingespeist werden.

Geeignete heterogene Katalysatorsysteme umfassen im allgemeinen eine der zuvor genannten Übergangsmetallverbindungen auf einem inerten Träger. Geeignete anorganische Träger sind die hierfür üblichen Oxide, insbesondere Silicium- und Aluminiumoxide, Alumosilikate, Zeolithe, Carbide, Nitride etc. und deren Mischungen. Bevorzugt werden als Träger Al₂O₃, SiO₂ und deren Mischungen verwendet. Insbesondere handelt es sich bei den im erfindungsgemäßen Verfahren verwendeten heterogenen Katalysatoren um die in den US-A-4,587,369; US-A-4,704,492 und US-A-4,493,906 beschriebenen, auf die hier in vollem Umfang Bezug genommen wird. Weiterhin geeignete Katalysatorsysteme auf Cu-Basis werden von der Fa. Dow Chemical als KLP-Katalysator vertrieben.

Bevorzugt werden für die selektive Hydrierung Olefinfraktionen eingesetzt, die einen Anteil an zwei- und mehrfach ungesättigten Verbindungen im Bereich von 60 bis 100 Gew.-% aufweisen.

### Schritt d)

Zur erfindungsgemässen Herstellung von Alkoholgemischen wird eine in Schritt b) isolierte und gegebenenfalls in Schritt c) selektivhydrierte Olefinfraktion hydroformyliert und hydriert. Dabei kann die Herstellung der Alkoholgemische einstufig oder in zwei separaten Reaktionsschritten erfolgen.

Bevorzugt weist die in Schritt d) eingesetzte Olefinfraktion eine Jodzahl im Bereich von 200 bis 400 g I₂/100 g auf.

Bevorzugt weist die in Schritt d) eingesetzte Olefinfraktion einen Anteil an unverzweigten Olefinen im Bereich von 10 bis 90 Gew.-%, bevorzugt 30 bis 70 Gew.-%, auf.

Geeignete Katalysatoren für die Hydroformylierung sind bekannt und umfassen im allgemeinen ein Salz oder eine Komplexverbindung eines Elementes der VIII. Nebengruppe des Periodensystems. Vorzugsweise ist das Metall der VIII. Nebengruppe ausgewählt unter Cobalt, Ruthenium, Iridium, Rhodium, Nickel, Palladium und Platin. Für das erfindungsgemäße Verfahren werden bevorzugt Salze und insbesondere Komplexverbindungen des Rhodiums oder des Cobalts verwendet.

Geeignete Salze sind beispielsweise die Hydride, Halogenide, Nitrate, Sulfate, Oxide, Sulfide oder die Salze mit Alkyl- oder Arylcarbonsäuren oder Alkyl- oder Arylsulfonsäuren. Geeignete Komplexverbindungen sind beispielsweise die Carbonylverbindungen und Carbonylhydride der genannten Metalle sowie Komplexe mit Aminen, Amiden, Triarylphosphinen, Trialkylphosphinen, Tricycloalkylphosphinen, Olefinen, oder Dienen als Liganden. Die Liganden können auch in polymerer oder polymergebundener Form eingesetzt werden. Auch können Katalysatorsysteme in situ aus den obengenannten Salzen und den genannten Liganden hergestellt werden.

Geeignete Alkylreste der Liganden sind die zuvor beschriebenen linearen oder verzweigten C₁-C₁₅-Alkyl, insbesondere C₁-C₅-Alkylreste. Cycloalkyl steht vorzugsweise für C₃-C₁₀-Cycloalkyl, insbesondere Cyclopentyl und Cyclohexyl, die gegebenenfalls auch mit C₁-C₄-Alkylgruppen substituiert sein können. Unter Aryl versteht man vorzugsweise Phenyl (Ph) oder Naphthyl, das gegebenenfalls mit 1, 2, 3 oder 4 C₁-C₄-Alkyl, C₁-C₄-Alkoxy, z.B. Methoxy, Halogen, vorzugsweise Chlorid, oder Hydroxy, das gegebenenfalls auch ethoxyliert sein kann, substituiert ist.

Geeignete Rhodiumkatalysatoren bzw. -katalysatorvorstufen sind Rhodium(II)- und Rhodium(III)salze wie Rhodium(III)chlorid, Rhodium(III)nitrat, Rhodium(III)sulfat, Kalium-Rhodiumsulfat (Rhodiumalaun), Rhodium(II)- bzw. Rhodium(III)carboxylat, vorzugsweise Rhodium(II)- und Rhodium(III)acetat, Rhodium(III)oxid, Salze der Rhodium(III)säure und Trisammoniumhexachlororhodat(III).

Weiterhin eignen sich Rhodiumkomplexe der allgemeinen Formel RhXₘL¹L²(L³)n, worin X für Halogenid, vorzugsweise Chlorid oder Bromid, Alkyl- oder Arylcarboxylat, Acetylacetonat, Aryl- oder Alkylsulfonat, insbesondere Phenylsulfonat und Toluolsulfonat, Hydrid oder das Diphenyltriazin-Anion,
L¹, L², L³ unabhängig voneinander für CO, Olefine, Cycloolefine, vorzugsweise Cyclooctadien (COD), Dibenzophosphol, Benzonitril, PR₃ oder R₂P-A-PR₂, m für 1, 2 oder 3 und n für 0, 1 oder 2 stehen. Unter R (die Reste R können gleich oder verschieden sein) sind Alkyl-, Cycloalkyl- und Arylreste zu verstehen, vorzugsweise Phenyl, p-Tolyl, m-Tolyl, p-Ethylphenyl, p-Cumyl, p-t.-Butylphenyl, p-C₁-C₄-Alkoxyphenyl, vorzugsweise p-Anisyl, Xylyl, Mesityl, p-Hydroxyphenyl, das gegebenenfalls auch ethoxyliert vorliegen kann, Isopropyl, C₁-C₄-Alkoxy, Cyclopentyl oder Cyclohexyl. A steht für 1,2-Ethylen oder 1,3-Propylen. Bevorzugt stehen L¹, L² oder L³ unabhängig voneinander für CO, COD, P(Phenyl)₃, P(i-Propyl)₃, P(Anisyl)₃, P(OC₂H₅)₃, P(Cyclohexyl)₃, Dibenzophosphol oder Benzonitril. X steht bevorzugt für Hydrid, Chlorid, Bromid, Acetat, Tosylat, Acetylacetonat oder das Diphenyltriazin-Anion, insbesondere für Hydrid, Chlorid oder Acetat.

Geeignete Cobaltverbindungen sind beispielsweise Cobalt(II)chlorid, Cobalt(II)sulfat, Cobalt(II)nitrat, deren Amin- oder Hydratkomplexe, Cobaltcarboxylate, wie Cobaltacetat, Cobaltethylhexanoat, Cobaltnaphthanoat, sowie die Carbonylkomplexe des Cobalts wie Dicobaltoctacarbonyl, Tetracobaltdodecacarbonyl und Hexacobalthexadecacarbonyl. Bevorzugt werden für das erfindungsgemäße Verfahren die Cobaltcarbonylkomplexe und insbesondere Dicobaltoctacarbonyl verwendet.

Die genannten Verbindungen des Rhodiums und Cobalts sind im Prinzip bekannt und in der Literatur hinreichend beschrieben oder sie können vom Fachmann analog zu den bereits bekannten Verbindungen hergestellt werden. Diese Herstellung kann auch in situ erfolgen, wobei die katalytisch aktive Spezies auch aus den zuvor genannten Verbindungen als Katalysatorvorstufen erst unter den Hydroformylierungsbedingungen gebildet werden kann.

Wird ein Hydroformylierungskatalysator auf Basis von Rhodium eingesetzt, so im Allgemeinen in einer Menge von 1 bis 150 ppm, bevorzugt bei 1 bis 100 ppm. Die Reaktionstemperatur liegt für einen Hydroformylierungskatalysator auf Basis von Rhodium im im Allgemeinen im Bereich von Raumtemperatur bis 200°C, bevorzugt 50 bis 170°C.

Wird ein Hydroformylierungskatalysator auf Basis von Cobalt eingesetzt, so im Allgemeinen in einer Menge von 0,0001 bis 0,5 Gew.-%, bezogen auf die Menge der zu hydroformylierenden Olefine. Die Reaktionstemperatur liegt für einen Hydroformylierungskatalysator auf Basis von Cobalt im im Allgemeinen im Bereich von etwa 100 bis 250°C, bevorzugt 150 bis 200°C.

Die Reaktion kann bei einem erhöhten Druck von etwa 10 bis 650 bar durchgeführt werden.

Das Molmengenverhältnis von H₂:CO beträgt im allgemeinen etwa 1:5 bis etwa 5:1.

Die bei der Hydroformylierung resultierenden Aldehyde bzw. Aldehyd/Alkohol-Gemische können vor der Hydrierung gewünschtenfalls nach üblichen, dem Fachmann bekannten Verfahren isoliert und gegebenenfalls gereinigt werden. Vorzugsweise wird vor der Hydrierung der Hydroformylierungskatalysator aus dem Reaktionsgemisch entfernt. Er kann im Allgemeinen, gegebenenfalls nach Aufarbeitung, erneut zur Hydroformylierung eingesetzt werden. Zur Hydrierung werden die bei der Hydroformylierung erhaltenen Reaktionsgemische mit Wasserstoff in Gegenwart eines Hydrierungskatalysators umsetzt.

Geeignete Hydrierungskatalysatoren sind im allgemeinen Übergangsmetalle wie z.B. Cr, Mo, W, Fe, Rh, Co, Ni, Pd, Pt, Ru, etc., oder deren Mischungen, die zur Erhöhung der Aktivität und Stabilität auf Trägern, wie Z.B. Aktivkohle, Aluminiumoxid, Kieselgur, etc., aufgebracht werden können. Zur Erhöhung der katalytischen Aktivität können Fe, Co, und bevorzugt Ni auch in Form der Raney-Katalysatoren als Metallschwamm mit einer sehr großen Oberfläche verwendet werden.

Bevorzugt wird für das erfindungsgemäße Verfahren ein Co/Mo-Katalysator eingesetzt.

Die Hydrierung der Oxo-Aldehyde erfolgt in Abhängigkeit von der Aktivität des Katalysators vorzugsweise bei erhöhten Temperaturen und erhöhtem Druck. Vorzugsweise liegt die Reaktionstemperatur bei etwa 80 bis 250°C. Bevorzugt liegt der Druck bei etwa 50 bis 350 bar.

Nach einer besonderen Ausführungsform des erfindungsgemäßen Verfahrens erfolgt die Herstellung der erfindungsgemässen Alkoholgemische in einer einstufigen Reaktion. Dazu wird eine Olefinfraktion mit Kohlenmonoxid und Wasserstoff in Gegenwart eines Hydroformylierungskatalysators, der auch für die weitere Hydrierung zu den Oxo-Alkoholen geeignet ist, umgesetzt. Allgemein sind alle Hydroformylierungskatalysatoren auch zur Durchführung katalytischer Hydrierungen geeignet, wobei jedoch in Abhängigkeit von der katalytischen Aktivität im allgemeinen höhere Temperaturen und/oder höhere Drücke und/oder längere Reaktionszeiten sowie eine größere Katalysatormenge als für eine ausschließliche Hydroformylierung verwendet werden.

Für das erfindungsgemäße Verfahren zur Hydroformylierung mit gleichzeitiger Hydrierung wird bevorzugt ein Cobaltcarbonyl-Katalysator und insbesondere Co₂(CO)₈ verwendet. Die Reaktionstemperatur liegt im allgemeinen bei 100 bis 220°C, bevorzugt bei 150 bis 200°C, bei einem erhöhten Druck von 50 bis 650 bar, bevorzugt 100 bis 600 bar.

Auch andere Verfahren können zur Reduktion der Aldehyde zu den Alkoholen verwendet werden. Dazu zählen z.B. die Reduktion mit komplexen Hydriden, wie z.B. LiAlH₄ und NaBH₄, die Reduktion mit Natrium in Ethanol nach Bouveault-Blanc sowie weitere bekannte Verfahren.

Ein weiterer Gegenstand der Erfindung sind die nach dem erfindungsgemässen Verfahren erhaltenen Alkoholgemische.

Vorzugsweise weisen die Alkoholgemische eine OH-Zahl im Bereich von etwa 300 bis 600 mg KOH/g Produkt, bevorzugt 350 bis 500 mg KOH/g Produkt, auf.

Vorzugsweise weisen die Alkoholgemische einen mittels NMR ermittelten Verzweigungsgrad im Bereich von 0,2 bis 2,0, bevorzugt 0,5 bis 1,5, auf.

Vorzugsweise findet bei der Hydrierung ein möglichst vollständiger Umsatz statt, so daß die Carbonylzahl der nach dem erfindungsgemäßen Verfahren erhaltenen Alkoholgemische im Allgemeinen gering ist. Im Allgemeinen weisen die erfindungsgemässen Alkoholgemische eine Carbonylzahl von höchstens 5 auf.

Die erfindungsgemässen Alkoholgemische eignen sich vorzugsweise für die Veresterung zur Herstellung von Ester-Weichmachern.

Ein weiterer Gegenstand der Erfindung ist ein Verfahren zur Herstellung eines Estergemisches, wobei man eines der zuvor beschriebenen erfindungsgemässen Alkoholgemische mit wenigstens einer Säure umsetzt, die ausgewählt ist unter aliphatischen Di- und Tricarbonsäuren, aromatischen Mono-, Di- und Tricarbonsäuren, Phosphorsäure und Derivaten und Mischungen davon.

Gegenstand der Erfindung ist somit ein Verfahren zur Herstellung von Estergemischen, wobei man
a) ein Kohlenwasserstoffgemisch, das Cyclopenten und wenigstens ein acyclisches Monoolefin enthält, in einer Metathesereaktion umsetzt,
b) aus dem Reaktionsgemisch der Metathese eine Olefinfraktion isoliert, die im Wesentlichen Olefine mit 6 bis 12 Kohlenstoffatomen enthält,
c) gegebenenfalls die in der Olefinfraktion enthaltenen zwei- oder mehrfach ungesättigten Verbindungen zumindest teilweise einer selektiven Hydrierung zu Monoolefinen unterwirft,
d) die, gegebenenfalls selektiv hydrierte, Olefinfraktion durch Umsetzung mit Kohlenmonoxid und Wasserstoff katalytisch hydroformyliert und hydriert, wobei ein Alkoholgemisch resultiert,
e) das Alkoholgemisch aus Schritt d) einer Veresterung mit wenigstens einer Säure oder einem Derivat davon unterwirft.

Bevorzugt ist die Säure ausgewählt unter aliphatischen C₅-C₁₅-Dicarbonsäuren, insbesondere Adipinsäure, Azelainsäure und Sebacinsäure, aliphatischen C₅-C₁₅-Tricarbonsäuren, insbesondere Citronensäure, aromatischen Monocarbonsäuren, insbesondere Benzoesäure, aromatischen Dicarbonsäuren, insbesondere Phthalsäure, Isophthalsäure und Terephthalsäure, aromatischen Tricarbonsäuren, insbesondere Trimellithsäure, Phosphorsäure und Derivaten und Gemischen davon. Vorzugsweise wird zur Herstellung des Estergemisches Phthalsäure oder ein Phthalsäurederivat eingesetzt.

Zur Veresterung geeignete Derivate der zuvor genannten aliphatischen und aromatischen Carbonsäuren sind z.B. Anhydride, Halogenide, wie Chloride, und Di-(C₁-C₄-alkyl)ester. Geeignete Phthalsäurederivate sind z.B. Phthalsäureanhydrid, Phthalsäurehalogenide, wie Phthalsäurechlorid, und Phthalsäuredi-(C₁-C₄-alkyl)ester. Geeignete Derivate der Phosphorsäure sind z.B. P₂O₅, Polyphosphorsäuren und Phosphorylhalogenide, wie Phosphorylchlorid.

Die Veresterung erfolgt nach üblichen, dem Fachmann bekannten Verfahren. Geeignete Verfahren zur Herstellung von Estern von Carbonsäuren und Carbonsäurederivaten sind z.B. im Organikum, VEB Deutscher Verlag der Wissenschaften, 16. Aufl. 1986, S. 402 ff. beschrieben. Werden zur Veresterung Di- und/oder Tricarbonsäuren oder deren Derivate eingesetzt, so werden die Säuregruppen bzw. derivatisierten Säuregruppen im Wesentlichen vollständig zu Estergruppen umgesetzt, die sich von den erfindungsgemässen Alkoholgemischen ableiten. Werden zur Veresterung Phosphorsäure oder deren Derivate eingesetzt, so erfolgt die Umsetzung im Wesentlichen zu Triestern, die sich von den erfindungsgemässen Alkoholgemischen ableiten. Vorzugsweise wird das erfindungsgemässe Alkoholgemisch mit einem molaren Überschuß von etwa 5 bis 30% an Säuregruppen oder derivatisierten Säuregruppen umgesetzt. Vorzugsweise erfolgt die Umsetzung in Gegenwart eines Acylierungskatalysators, wie eines Dialkyltitanats, z.B. Isopropylbutyltitanat, oder einer Säure, wie Methansulfonsäure, Toluolsulfonsäure oder Schwefelsäure.

Die Umsetzung mit einer aliphatischen Carbonsäure oder einem Carbonsäurederivat findet im Allgemeinen bei Reaktionstemperaturen von 60 bis 200°C statt. Die Umsetzung mit einer aromatischen Carbonsäure oder einem Carbonsäurederivat, wie z.B. die Umsetzung mit Phthalsäure oder einem Phthalsäurederivat, findet im Allgemeinen bei Reaktionstemperaturen von 150 bis 250°C, vorzugsweise 200 bis 250°C statt. Bei einer geeigneten Ausführungsform wird während der Umsetzung ein Inertgas, wie Stickstoff, in das Reaktionsgemisch eingeperlt und das gebildete Reaktionswasser fortlaufend mit dem Inertgasstrom aus dem Reaktionsgemisch entfernt. Nach beendeter Umsetzung wird aus dem Reaktionsgemisch das erfindungsgemässe Estergemisch isoliert. Dies kann z.B. durch Abtrennen von gegebenenfalls nicht umgesetzten Edukten, z.B. durch Destillation im Vakuum, erfolgen. Das rohe Estergemisch kann mit einer wässrigen Lauge, z.B. Natronlauge, neutralisiert werden. Dabei resultiert im Allgemeinen ein Zweiphasengemisch, aus dem die organische Phase separiert und anschliessend gewünschtenfalls weiteren Waschschritten unterzogen werden kann. Zur weiteren Reinigung kann das Estergemisch vorzugsweise mit Wasserdampf bei erhöhter Temperatur ausgedämpft werden. Das gereinigte Estergemisch kann dann bei erhöhter Temperatur im Vakuum durch Durchleiten eines Inertgasstroms getrocknet, und gegebenenfalls durch Inkontaktbringen mit einem Adsorptionsmittel, wie Aktivkohle oder Bleicherde, weiter gereinigt werden.

Die erfindungsgemässen Estergemische eignen sich bevorzugt als Weichmacher oder als Komponente von Weichmachergemischen. Vorzugsweise eignen sie sich als Weichmacher für Formmassen, insbesondere Formmassen auf PVC-Basis. Bevorzugt werden Phthalsäurediester auf Basis der erfindungsgemässen Alkoholgemische eingesetzt. Vorzugsweise zeichnen sich die erfindungsgemässen Estergemische durch eine besonders niedrige Lösetemperatur für PVC (Polyvinylchlorid) und damit durch ein besonders gutes Geliervermögen aus. Vorteilhafterweise zeigen die erfindungsgemässen Estergemische eine sehr gute Verträglichkeit in Weich-PVC-Compounds. Die Verträglichkeit eines Weichmachers in einem Weich-PVC-Compound wird z.B. durch Lagerung des Compound bei einer erhöhten Temperatur, wie etwa 70°C, und erhöhter relativer Luftfeuchtigkeit, wie etwa 100%, über einen längeren Zeitraum bestimmt, wobei der Gewichtsverlust des Compound infolge des Ausschwitzens von Weichmacher nach bestimmten Zeitintervallen durch Auswiegen ermittelt wird. Je grösser der Gewichtsverlust ist, desto geringer ist die Verträglichkeit des Compound mit dem Weichmacher. Vorteilhafterweise ist der Gewichtsverlust eines Weich-PVC-Compounds auf Basis eines erfindungsgemässen Estergemisches als Weichmacher im Allgemeinen geringer als der eines Weich-PVC-Compounds auf Basis von kommerziell erhältlichen Weichmachern.

Ein weiterer Gegenstand der Erfindung ist eine Polymerzusammensetzung, enthaltend:
i) wenigstens ein Polymer, das ausgewählt ist unter Acrylharzen, Polyamiden, Polyethylenterephthalat, Polyolefinen, Polystyrol, Fluorpolymeren, PVC-Homo- und Copolymeren und Mischungen davon,
ii) wenigstens ein erfindungsgemässes Estergmisch,
iii) gegebenenfalls weitere Zusatzstoffe.

### Komponente i)

Vorzugsweise enthalten die erfindungsgemässen Polymerzusammensetzungen eine Polymerkomponente, die ausgewählt ist unter Acrylharzen, bevorzugt Polymethylmethacrylat, Polyamiden, Polyethylenterephthalat, Polyolefinen, bevorzugt Polypropylen, Polystyrol, Fluorpolymeren, PVC-Homo- und Copolymeren und Mischungen davon. Vorzugsweise enthält die Polymerkomponente PVC und Polymergemische, die PVC enthalten.

Vorzugsweise wird zur Herstellung der erfindungsgemässen Polymerzusammensetzungen pulverförmiges PVC eingesetzt. Insbesondere handelt es sich um pulverförmiges PVC das nach dem Suspensionsverfahren hergestellt wurde. Geeignete Polymerzusammensetzungen auf Basis von PVC-Pulvern und Weichmachern sind dem Fachmann als Plastisole bekannt.

Bevorzugt enthalten die erfindungsgemässen Polymerzusammensetzungen die Polymerkomponente i) in einer Menge von 50 bis 80 Gew.-%, bezogen auf die Gesamtmenge der Zusammensetzung.

### Komponente ii)

Die erfindungsgemässen Polymerzusammensetzungen enthalten wenigstens ein erfindungsgemässes Estergemisch, wie zuvor beschrieben. Vorzugsweise handelt es sich um ein Phthalsäurediestergemisch.

Bevorzugt enthalten die erfindungsgemässen Polymerzusammensetzungen die Komponente ii) in einer Menge von 20 bis 50 Gew.-%, bezogen auf die Gesamtmenge der Zusammensetzung.

### Komponente iii)

Die erfindungsgemässen Polymerzusammensetzungen können zusätzlich zu den zuvor genannten Komponenten wenigstens einen weiteren Zusatzstoff enthalten. Bevorzugt ist die Komponente iii) ausgewählt unter Stabilisatoren, Füllstoffen, Pigmenten, Farbstoffen, Flamminhibitoren, Lichtstabilisatoren, Antistatika, Treibmitteln, Biostabilisatoren etc.

Bevorzugt enthalten die erfindungsgernässen Polymerzusammensetzungen die Komponente(n) iii) in einer Menge von 0,1 bis 50 Gew.-%, bevorzugt 0,5 bis 45 Gew.-%, bezogen auf die Gesamtmenge der Zusammensetzung.

Die Herstellung der erfindungsgemässen Polymerzusammensetzungen erfolgt nach üblichen, dem Fachmann bekannten Verfahren. Dazu wird z.B. ein Gemisch aus Polymerkomponente, z.B. PVC-Pulver, dem erfindungsgemässen Estergemisch als Weichmacher sowie ggf. weiteren Zusätzen hergestellt. Dieses Gemisch wird anschliessend nach üblichen Verfahren plastifiziert, z.B. auf einem Mischwalzwerk. Nach dem Plastifizieren können sich weitere Bearbeitungsschritte anschliessen, wie Z.B. Walzen und Pressen.

Die erfindungsgemässen Polymerzusammensetzungen, die ein erfindungsgemässes Estergemisch als Weichmacher enthalten zeichnen sich durch gute anwendungstechnische Eigenschaften aus.

Die Erfindung wird anhand der folgenden nicht einschränkenden Beispiele erläutert.

### Beispiele

Für die Aufnahme der Gaschromatogramme wurde ein 5890 Gaschromatograph der Fa. Hewlett Packard mit einer DB 5,30 m x 0,32 mm Glaskapillarsäure und einem Flammionisationsdetektor mit angeschlossener Integriereinheit verwendet.

Die Iodzahl ist definiert als g Iod/100 g Produkt und wurde ermittelt nach Kaufmann. Dazu werden in einen 300 ml Erlenmeyerkolben ca. 0,2 g Prüfsubstanz exakt eingewogen, in 20 ml Chloroform gelöst, mit genau 20,00 ml Brom-Lösung versetzt und 2 Stunden im Dunkeln stehen gelassen. Danach werden 10 ml Kaliumiodid-Lösung und ca. 2 g Kaliumiodat hinzugegeben. Das ausgeschiedene Iod wird mit Natriumthiosulfatmaßlösung gegen Stärkelösung bis zum Verschwinden der Blaufärbung titriert. Zur Herstellung der verwendeten Bromlösung nach Kaufmann werden 120 g Natriumbromid in ca. 900 ml Methanol gelöst. Hierzu gibt man 6,5 ml Brom und füllt mit Methanol auf 1000 ml auf. Die Lösung ist dann ca. 0,25 molar und wird in braunen Glasflaschen aufbewahrt.

Die Alkoholzahl ist definiert als mg KOH/g Produkt. Zur Bestimmung werden ca. 1 g Prüfsubstanz exakt eingewogen, 9,8 ml Acetylierungsreagenz hinzugefügt und 24 Stunden bei Raumtemperatur stehen gelassen. Danach werden 25 ml dest. Wasser zugefügt und 15 min. gerührt, 25 ml Isopropanol zugesetzt und mit Natronlauge-Maßlösung gegen den Wendepunkt potentiometrisch titriert. Zur Herstellung des Acetylierungsreagenzes werden 810 ml Pyridin, 100 ml Essigsäureanhydrid und 9 ml Essigsäure vermischt.

### I. Metathese

### Beispiel 1:

Ein 1:1-Gemisch aus je 17,1 mol Cyclopenten und 1-Penten wurde bei Raumtemperatur und Normaldruck mit einer "in situ"-erzeugten Katalysatormischung aus 8,6 mmol (p-Cymol)RuCl₂(PCy₃) und 2 ml Me₃SiCHN₂ in 50 ml CH₂Cl₂ versetzt. Hierbei wurde eine schwache Gasentwicklung beobachtet. Nach 3 Stunden Rühren wurde die Lösung über neutrales Al₂O₃ chromatographiert und das farblose Filtrat destillativ von nicht umgesetzten Leichtsiedern befreit. Es verblieben 956 g einer farblosen, niederviskosen Flüssigkeit nachfolgender Zusammensetzung (GC-Flächenprozent):
26% C₁₀H₁₈, 22% C₁₅H₂₆, 17% C₂₀H₃₄, 13% C₂₅H₄₂, 10% C₃₀H₅₀, 7% C₃₅H₅₈, 5% C₄₀H₆₆.
Jodzahl: 351 g J₂/100 g

### Beispiel 2:

1 l C₅-Schnitt (Cyclopenten-Gehalt:15%) wurde bei Raumtemperatur unter Normaldruck mit einer Lösung von 0,6 mmol RuCl₂(=CHPh)(PCy₃)₂ in 20 ml CH₂Cl₂ umgesetzt. Hierbei wurde eine schwache Gasentwicklung beobachtet. Nach 1 h Rühren wurde die Lösung über Al₂O₃ chromatographiert und das farblose Filtrat destillativ von nicht umgesetzten Leichsiedern befreit. Man erhielt 96 g einer farblosen, niederviskosen Flüssigkeit folgender Zusammensetzung (GC-Flächenprozent):
4% C₇H₁₂, 11% C₈H₁₆, 14% C₁₀H₁₈, 3% C₁₂H₂₀, 8% C₁₃H₂₄, 12% C₁₅H₂₆, 2% C₁₇H₂₈, 5% C₁₈H₃₂, 9% C₂₀H₃₄, 1% C₂₂H₃₆, 4% C₂₃H₄₀, 7% C₂₅H₄₂, 3% C₂₈H₄₈, 6% C₃₀H₅₀, 1% C₃₃H₅₆, 4% C₃₅H₅₈, 3% C₄₀H₅₈, 3% C₄₀H₆₆, 2% C₄₀H₆₆, 1% C₄₀H₆₆.
Jodzahl: 329 g J₂/100 g

### Beispiel 3:

Ein 1:1-Gemisch aus Cyclopenten und 1-Penten wurde bei 60°C, 5 bar und Verweilzeiten von 1-3 h kontinuierlich in einen mit ReO₇/Al₂O₃ bestückten Rohrreaktor gepumpt. Mit Hilfe eines bei 115°C und Normaldruck betriebenen Fallfilmverdampfers wurde das Reaktionsprodukt anschließend in eine Leicht- und eine Hochsiederfraktion getrennt und erstere in den Metatheseprozeß zurückgeführt. Die Hochsiederfraktion wurde im Vakuum von Restmengen an Leichtsiedern befreit. Man erhielt bei Raum-Zeit-Ausbeuten von 50-500 g l⁻¹ h⁻¹ leicht gelbliche Flüssigkeiten, die abschließend über Al₂O₃ chromatographiert wurden. Eine entnommene Probe hatte folgende Zusammensetzung (GC-Flächenprozent):
3% C₇H₁₂, 9% C₈H₁₆, 16% C₁₀H₁₈, 2% C₁₂H₂₀, 8% C₁₃H₂₄, 13% C₁₅H₂₆, 2% C₁₇H₂₈, 6% C₁₈H₃₂, 11% C₂₀H₃₄, 1% C₂₂H₃₆, 4% C₂₃H₄₀, 9% C₂₅H₄₂, 2% C₂₈H₄₈, 6% C₃₀H₅₀, 3% C₃₅H₅₈, 2% C₄₀H₆₆, 1% C₄₀H₆₆, 1% C₄₅H₇₄.
Jodzahl: 349 g J₂/100 g

### Beispiel 4:

1 l C₅-Schnitt wurde bei 60°C, 5 bar und einer Verweilzeit von 1,2 h kontinuierlich in einen mit Re₂O₇/Al₂O₃ bestückten Rohrreaktor gepumpt. Mit Hilfe eines bei 115°C und Normaldruck betriebenen Fallfilmverdampfers wurde das Reaktionsprodukt in eine Leicht- und eine Hochsiederfraktion getrennt. Letztere wurde durch Destillation im Vakuum von Restmengen an Leichtsiedern befreit. Man erhielt bei Raum-Zeit-Ausbeuten von 85 g l⁻¹ h⁻¹ und CyclopentenUmsätzen bis 70% eine leicht gelbliche Flüssigkeit, die abschließend über Al₂O₃ chromatographiert wurde. Eine entnommene Probe hatte folgende Zusammensetzung (GC-Flächenprozent):
47% isomere C₆H₁₂, C₇H₁₂, C₇H₁₄, C₈H₁₄, C₈H₁₆, C₉H₁₆, C₉H₁₈, 44% isomere C₁₀H₁₈, C₁₁H₂₀, C₁₂H₂₀, C₁₂H₂₂, C₁₃H₂₂, C₁₃H₂₄, C₁₄H₂₄, C₁₄H₂₆,
9% isomere C₁₅H₂₆ ₋ C₂₅H₄₂.
Jodzahl: 325 g J₂/100 g

### II. Alkoholherstellung

### Beispiel 5

Ein Metathese-Reaktionsgemisch gemäss Beispiel 4 wurde einer fraktionierten Destillation in einer 90 cm Füllkörperkolonne unterzogen (Fraktion isoliert bei 1013 mbar, 100 °C bis 200 mbar, 102 °C, Sumpftemperatur 130 °C). Dabei wurde eine Olefinfraktion folgender Zusammensetzung (GC-Flächenprozent) isoliert:
43,7% C₇-, 23,7% C₈-, 15,2% C₉-, 7,5% C₁₀-Olefine. Die Jodzahl betrug 231 g J₂/100 g.
500 g dieser Olefinfraktion wurden mit 1,46 g Co₂(CO)₈ bei 185°C und 280 bar mit Synthesegas (CO/H₂ 1:1) unter Zusatz von 50 g Wasser und 500 g n-Hexan in einem 2,5 l Drehrührautoklaven hydroformyliert, die Reaktionszeit betrug 5 Stunden. Nach Abkühlen und Entspannen des Autoklaven wurde der Reaktionsaustrag mit 10%iger Essigsäure unter Lufteinleitung bei 90°C entcobaltet. Das resultierende Hydroformylierungsgexnisch wurde in einem 2,5 l Rohrreaktor in Rieselfahrweise an einem Co/Mo-Festbettkatalysator bei 175°C und 280 bar mit Wasserstoff unter Zusatz von 10 Gew.-% Wasser hydriert.

Das erhaltene Alkoholgemisch wurde destillativ aufgearbeitet und eine Fraktion von 660g mit einem Siedebereich von 100 °C/25 mbar bis 99°C/9 mbar isoliert. Diese Fraktion hat eine OH-Zahl von 413 mg KOH/g. Mittels ¹H-NMR wurde ein mittlerer Verzweigungsgrad von 0,8 ermittelt.

### Beispiel 6 (Phthalsäurediesterherstellung)

865,74 g des in Beispiel 5 erhaltenen Alkoholgemisches wurden mit 370,30 g Phthalsäureanhydrid und 0,42 g Isopropylbutyltitanat als Katalysator in einem 2l-Autoklaven unter Stickstoffeinperlung (10 l/h) bei einer Rührgeschwindigkeit von 550 U/min und einer Reaktionstemperatur von 230°C umgesetzt. Das gebildete Reaktionswasser wurde fortlaufend mit dem Stickstoffstrom aus dem Reaktionsgemisch entfernt. Die Reaktionszeit betrug 180 min. Anschliessend wurde der überschüssige Alkohol bei einem Vakuum von 50 mbar abdestilliert. 1000 g des Rohestergemisches wurden mit 150 ml 0,5%iger Natronlauge durch 10minütiges Rühren bei 80°C neutralisiert. Es bildete sich ein Zwei-Phasen-Gemisch mit einer oberen organischen und einer unteren wässrigen Phase (Ablauge mit hydrolysiertem Katalysator). Die wässrige Phase wurde abgetrennt und die organische Phase zweimal mit je 200 ml Wasser nachgewaschen. Zur weiteren Reinigung wurde das neutralisierte und gewaschene Estergemisch mit Wasserdampf bei 180°C und 50 mbar Vakuum 2 h ausgedämpft. Das gereinigte Estergemisch wurde dann 30 min bei 150°C/50 mbar mittels Durchleiten eines Stickstoffstroms (2 l/h) getrocknet, anschliessend mit Aktivkohle 5 min verrührt und bei etwa 80°C über eine Nutsche mit Filterhilfsmittel Supra-Theorit® 5 abgesaugt.

Das so erhaltene Estergemisch besitzt eine Dichte von 1,022 g/cm³, eine Viskosität von 308 mPa s, einen Brechungsindex n_{D}²⁰ von 1,5018, eine Säurezahl von 0,03 mg KOH/g, einen Wassergehalt von 0,03% und eine gaschromatographisch ermittelte Reinheit von 99,7%. Die Lösetemperatur für PVC beträgt 112°C.

### Anwendungstechnische Beispiele

Die Lösetemperatur für PVC wird nach DIN 53408 bestimmt.

Die Herstellung von Weich-PVC-Compounds unter Einsatz der erfindungsgemässen Estergemische und deren Verträglichkeitsprüfung erfolgt nach der folgenden allgemeinen Methode:

Zunächst wird ein Gemisch aus PVC-Pulver (bevorzugt hergestellt nach dem Suspensionsverfahren), dem erfindungsgemässen Estergemisch als Weichmacher sowie ggf. weiteren Zusätzen, wie Stabilisatoren, Gleitmitteln, Füllstoffen, Pigmenten, Farbstoffen, Flamminhibitoren, Lichtstabilisatoren, Antistatika, Treibmitteln, Biostabilisatoren etc. hergestellt. Dieses Gemisch wird anschliessend auf einem Mischwalzwerk plastifiziert und zu einem sogenannten Walzfell gewalzt. Das Walzfell wird anschliessend zu einer Weich-PVC-Folie verpresst, an der dann die anwendungstechnischen Untersuchungen durchgeführt werden.

### Verträglichkeitsprüfung

Die Verträglichkeit eines Weichmachers in einem Weich-PVC-Compound wird durch Lagerung des Compound bei einer Temperatur von 70°C und 100% relativer Luftfeuchtigkeit über einen längeren Zeitraum bestimmt, wobei der Gewichtsverlust des Compound infolge des Ausschwitzens von Weichmacher nach bestimmten Zeitintervallen durch Auswiegen ermittelt wird.

Zur Prüfug werden Prüfkörper (Folien) mit einer Grösse von 75 x 110 x 0,5 mm eingesetzt.

Im Innenraum eines auf 70°C temperierten Trockenschrankes werden die gewogenen Folien auf ein Drahtgestell gehängt und in eine Glaswanne gestellt, die ca. 5 cm hoch mit vollentsalztem Wasser gefüllt ist. Dabei berühren die Folien sich weder gegenseitig noch den Wasserspiegel. Die Wanne wird mit Polyethylenfolie wasserdampfdicht verschlossen, um ein Entweichen von entstehendem Wasserdampf zu verhindern. Nach Bedarf wird Wasser ersetzt.

Im Tages-Rhythmus werden jeweils zwei Folien der Glaswanne entnommen und eine Stunde an der Luft frei hängend klimatisiert. Danach werden die Folien mit Methanol oberflächlich gereinigt und 16 h bei 70°C in einem Trockenschrank mit Zwangskonvektion frei hängend getrocknet. Nach Entnahme aus dem Trockenschrank werden die Folien erneut eine Stunde an der Luft frei hängend klimatisiert und anschliessend gewogen. Aus dem Gewichtsverlust der Folien wird der arithmetische Mitelwert gebildet.

### Beispiel 7

### Herstellung und Prüfung eines Weich-PVC-Compounds unter Einsatz eines erfindungsgemässen Estergemisches

100 g Suspensions-PVC vom Typ Vinoflex® S 7114 der BASF AG, 67 g des erfindungsgemässen Estergemisches aus Beispiel 6 und 2 g Ba/Zn-Stabilisator vom Typ Lankromark® LZB 753 werden mit einem Handmixer bei Raumtemperatur vermischt. Die Mischung wird anschliessend auf einem dampfbeheizten Labormischwalzwerk (Fa. Collin, Typ 150) plastifiziert und zu einem Walzfell verarbeitet. Die Temperatur der beiden Walzen beträgt jeweils 170°C, die Drehzahlen liegen bei 15 U/min (vordere Walze) und 12 U/min (hintere Walze), die Walzzeit beträgt 5 min. Man erhält ein Walzfell mit einer Dicke von 0,55 mm. Das abgekühlte Walzfell wird anschliessend bei einer Temperatur von 180°C und einem Druck von 220 bar innerhalb von 400 s auf einer Presse vom Typ 400 P der Fa. Collin zu einer Weich-PVC-Folie mit einer Dicke von 0,50 mm verpresst. Diese Folie wird zur Verträglichkeitsprüfung nach obiger Vorschrift eingesetzt. Die Ergebnisse sind in Tabelle 1 wiedergegeben.

### Vergleichsbeispiele 8 bis 10

Analog zu Beispiel 7 wurde die Lösetemperatur für PVC und die Verträglichkeit von drei kommerziell verfügbaren Phthalsäureester-Weichmachern bestimmt. Die Ergebnisse sind ebenfalls in Tabelle 1 wiedergegeben.

**Tabelle 1**

| Beispiel Nr. | 7 | 8 (Vergleich) | 9 (Vergleich) | 10 (Vergleich) |
|---|---|---|---|---|
| Weichmacher | Phthalsäurediester aus Bsp. 6 | Di-(2-ethylhexyl)-phthalat | Diisononylphthalat | Diisodecylphthalat |
| Lösetemperatur für PVC (°C) | 112 | 124 | 130 | 140 |
| Gewichtsverlust des Weich-PVC-Compounds (Gew.-%) | 0,07 (1 d) | 0,15 | 0,14 | 0,23 |
| | 0,12 (2 d) | 0,21 | 0,23 | 0,24 |
| | 0,24 (3 d) | 0,32 | 0,37 | 0,41 |

Wie die Daten in Tabelle 1 eindeutig belegen, weist das erfindungsgemässe Estergemisch eine deutlich niedrigere Lösetemperatur für PVC auf als die kommerziell erhältlichen Phthalsäureester und besitzt somit ein deutlich besseres Geliervermögen. Darüberhinaus zeigt das erfindungsgemässe Estergemisch in Weich-PVC-Compounds eine signifikant bessere Verträglichkeit des Weichmachers gegenüber den kommerziell erhältlichen Phthalsäureestern.

## Patentansprüche

1. Verfahren zur Herstellung von Alkoholgemischen, wobei man
a) ein Kohlenwasserstoffgemisch, das Cyclopenten und wenigstens ein acyclisches Monoolefin enthält, in einer Metathesereaktion umsetzt,
b) aus dem Reaktionsgemisch der Metathese eine Olefinfraktion isoliert, die im Wesentlichen Olefine mit 6 bis 12 Kohlenstoffatomen enthält,
c) gegebenenfalls die in der Olefinfraktion enthaltenen zwei- oder mehrfach ungesättigten Verbindungen zumindest teilweise einer selektiven Hydrierung zu Monoolefinen unterwirft, und
d) die, gegebenenfalls selektiv hydrierte, Olefinfraktion durch Umsetzung mit Kohlenmonoxid und Wasserstoff katalytisch hydroformyliert und hydriert.

2. Verfahren nach Anspruch 1, wobei man in Schritt a) ein Kohlenwasserstoffgemisch mit einem Cyclopentengehalt im Bereich von 5 bis 40 Gew.-%, bevorzugt 10 bis 30 Gew.-%, einsetzt.

3. Verfahren nach einem der Ansprüche 1 oder 2, wobei man in Schritt b) eine Olefinfraktion isoliert, die im Wesentlichen Olefine mit 7 bis 11 Kohlenstoffatomen, bevorzugt 8 bis 10 Kohlenstoffatomen, enthält.

4. Verfahren nach einem der vorhergehenden Ansprüche, wobei die in Schritt d) eingesetzte Olefinfraktion eine Jodzahl im Bereich von 200 bis 400 g I₂/100 g aufweist.

5. Verfahren nach einem der vorhergehenden Ansprüche, wobei die in Schritt d) eingesetzte Olefinfraktion einen Anteil an unverzweigten Olefinen im Bereich von 10 bis 90 Gew.-%, bevorzugt 30 bis 70 Gew.-%, aufweist.

6. Alkoholgemisch, erhältlich durch ein Verfahren nach einem der Ansprüche 1 bis 5.

7. Alkoholgemisch nach Anspruch 6, das eine OH-Zahl im Bereich von 300 bis 600 mg KOH/g Produkt, bevorzugt 350 bis 500 mg KOH/g, aufweist.

8. Alkoholgemisch nach einem der Ansprüche 6 oder 7, das einen mittleren Verzweigungsgrad im Bereich von 0,2 bis 2,0, bevorzugt 0,5 bis 1,5, aufweist.

9. Verfahren zur Herstellung eines Estergemisches, wobei man ein Alkoholgemisch nach einem der Ansprüche 6 bis 8 mit wenigstens einer Säure, die ausgewählt ist unter aliphatischen Di- und Tricarbonsäuren, aromatischen Mono-, Di- und Tricarbonsäuren, Phosphorsäure und Derivaten und Mischungen davon, umsetzt.

10. Verfahren nach Anspruch 9, wobei die Säure ausgewählt ist unter Adipinsäure, Azelainsäure, Sebacinsäure, Citronensäure, Benzoesäure, Phthalsäure, Isophthalsäure, Terephthalsäure, Trimellithsäure, Phosphorsäure und Derivaten und Mischungen davon.

11. Estergemisch, erhältlich durch ein Verfahren nach einem der Ansprüche 9 oder 10.

12. Verwendung der Estergemische nach Anspruch 11 als Weichmacher oder als Komponente eines Weichmachergemisches, bevorzugt für Formmassen, insbesondere für Formmassen auf PVC-Basis.

13. Polymerzusammensetzung, enthaltend:
i) wenigstens ein Polymer, das ausgewählt ist unter Acrylharzen, Polyamiden, Polyethylenterephthalat, Polyolefinen, Polystyrol, Fluorpolymeren, PVC-Homo- und Copolymeren und Mischungen davon,
ii) wenigstens ein Estergmisch nach Anspruch 11,
iii) gegebenenfalls weitere Zusatzstoffe.
